Europäisches Patentamt

European Patent Office

Office européen des brevets

(19)

(11) Numéro de publication : **0 408 628 B1**

(12) **FASCICULE DE BREVET EUROPEEN**

(45) Date de publication du fascicule du brevet :
08.07.92 Bulletin 92/28

(21) Numéro de dépôt : **89904091.9**

(22) Date de dépôt : **24.03.89**

(86) Numéro de dépôt international :
**PCT/FR89/00137**

(86) Numéro de dépôt international :
**PCT/FR89/00137**

(87) Numéro de publication internationale :
**WO 89/09076 05.10.89 Gazette 89/24**

(51) Int. Cl.⁵ : **A61M 5/32**

(54) **SERINGUE DE SECURITE POUR PRISES DE SANG ET INJECTIONS.**

(30) Priorité : **29.03.88 FR 8804410**

(43) Date de publication de la demande :
**23.01.91 Bulletin 91/04**

(45) Mention de la délivrance du brevet :
**08.07.92 Bulletin 92/28**

(84) Etats contractants désignés :
**AT BE CH DE FR GB IT LI LU NL SE**

(56) Documents cités :
**DE-U- 8 800 767**
**FR-A- 2 243 705**
**GB-A- 1 096 439**
**US-A- 3 658 061**
**US-A- 3 884 230**

(73) Titulaire : **brunet, Jean-Louis**
**51-53, rue du Commandant-Charcot**
**F-69110 Sainte-Foy-les-Lyon (FR)**

(72) Inventeur : **brunet, Jean-Louis**
**51-53, rue du Commandant-Charcot**
**F-69110 Sainte-Foy-les-Lyon (FR)**

(74) Mandataire : **Monnier, Guy et al**
**Cabinet Monnier 150 Cours Lafayette B.P.**
**3058**
**F-69393 Lyon Cédex 03 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention se rapporte à un dispositif technique perfectionné pour prises de sang et injections, visant conjointement en premier lieu à protéger les utilisateurs des accidents possibles par piqure ou blessure avec l'aiguille souillée, et en deuxième lieu à permettre un reflux du sang par l'aiguille lors de sa pénétration dans la veine, apportant ainsi par un même mécanisme deux fonctions inexistantes dans les système de prises de sang utilisant des tubes sous vide pour la collecte d'échantillons sanguins multiples. On notera par ailleurs un troisièpe avantage apporté par l'invention, représenté par le fait que l'aiguille intra-veineuse se trouve parallèle au plan de peau lorsque le système est présenté en position de pénétration de l'aiguille sur le bras avant la piqûre, alors que dans le système existant, l'aiguille présente avec le plan de peau une angulation souvent gênante pour l'utilisateur qui veut effectuer une prise de sang.

La première fonction, dite de sécurité, en protégeant l'utilisateur du risque de blessure par l'aiguille souillée est fondamentale, étant donné le risque actuel représenté par les maladies transmissibles par le sang.

La conception du système selon l'invention permet par le même mécanisme la présentation parallèle au plan de peau de l'aiguille lors de la prise de sang et le reflux visualisé du sang dans l'aiguille (2ème fonction) lors de sa pénétration intra-veineuse, donnant ainsi à l'opérateur un moyen très facilité d'effectuer ses prélèvement sans aucun risque de piqûre et de contact avec le sang, la position de la main tenant le système étant très naturelle.

On rappelera que le document US-A-3 658 061 J.P. HALL décrit un dispositif de protection appliqué à une aiguille destinée à la pose d'un cathéter, laquelle aiguille, après que le cathéter ait été mis en place et qu'elle ait été retirée de la veine, reste autour dudit catheter sur le corps du patient aussi longtemps que ce cathéter est maintenu en fonction. Pour immobiliser l'aiguille et recouvrir sa pointe en vue d'éviter tout risque de blessure du patient, il est prévu, selon le document précité, un protecteur déformable qui présente une rainure dans laquelle vient s'scamoter l'aiguille.

Le dispositif suivant la présente invention est défini à la revendication 1.

En fait le dispositif suivant l'invention comporte, comme dans la technique antérieure, une aiguille propre à s'escamoter dans la rainure d'un protecteur, mais ce dernier et l'aiguille sont montés sur un manchon droit, souple et élastique, fixé avec le protecteur à la partie antérieure du support de tubes à vide. Ce manchon, grâce à sa souplesse et son élasticité, va permettre de passer, avec un mouvement angulaire-curviligne provoqué par une pression de l'index de l'opérateur, de la position de protection de l'aiguille à la position de pénétration intraveineuse, le retour à la position de sécurité se faisant par son élasticité.

Le reflux du sang dans le système après pénétration de l'aiguille en intra-veineuse est obtenu par la création d'un vide à l'intérieur du manchon avant pénétration intra-veineuse de l'aiguille, lorsque l'opérateur, venant de piquer, relâche le manchon qu'il avait pressé pour amener l'aiguille en position de pénétration dans la veine, ayant ainsi chassé au préalable une partie de l'air contenu dans ce manchon. Le sang va se trouver aspiré dans le manchon qui étant translucide, va montrer à l'opérateur que l'aiguille est bien positionnée dans la veine de son patient.

Le dispositif selon l'invention est adaptable aux systèmes de prélèvement de sang utilisant des tubes à vide, mais également aux seringues classiques.

La figure 1 est une coupe longitudinale représentant le dispositif selon l'invention en position de sécurité.

La figure 2 représente ce dispositif en position de pénétration de l'aiguille.

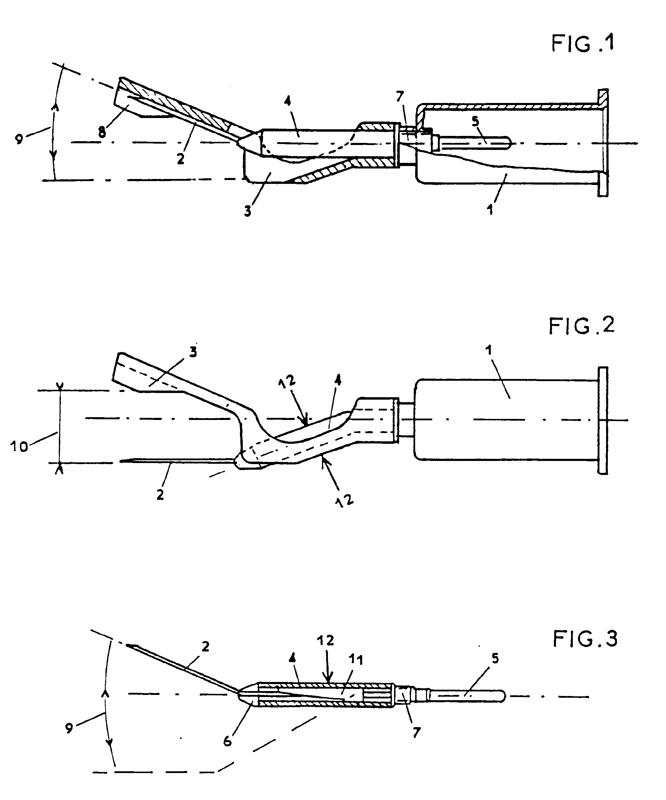

Le dispositif représente sur la figure 1 comporte le support de récipients sous vide (1) assemblé au protecteur d'aiguille (3) par un filetage (7). Le protecteur (3) porte à son extrémité une rainure (8) dans laquelle vient se loger la pointe de l'aiguille (2).

La figure 3 montre l'aiguille (2) montée sur son embase (6) formant un angle d'environ 155° avec l'axe du manchon droit élastique (4).

Dans une réalisation, c'est l'aiguille qui est incurvée à sa base ; dans une autre réalisation, c'est l'embase qui est incurvée, l'aiguille étant droite.

Cette figure 3 montre également la chambre de contrôle (11) à l'intérieur du manchon droit élastique (4) où se produit le reflux du sang, et le système (5) de perforation des récipients sous vide comportant une aiguille orientée en sens inverse et recouverte d'un capuchon souple.

La figure 3 montre enfin le mouvement angulaire-curviligne selon (9) du manchon souple élastique droit (4) obtenu par la pression de l'index de l'opérateur selon (12) ; le relâchement antagoniste de cette pression lorsque l'aiguille est en intra-veineuse provoque l'aspiration du sang à l'intérieur du manchon, en prouvant la situation correcte de l'aiguille dans la veine du patient. Si le sang n'apparaît pas dans la chambre (11), c'est que l'aiguille n'est pas correctement engagée dans la veine.

Lorsque la quantité de sang nécessaire a été prélevée, il suffit de retirer l'aiguille de la veine. Dès la sortie de la pointe de l'aiguille de la veine, celle-ci va retourner à sa position initiale dans le rainurage du protecteur grâce au manchon qui par son élasticité revient immédiatement à sa position droite de façon automatique, sans aucune manipulation supplémentaire de l'opérateur.

La caractéristique du dispositif selon l'invention est d'être d'une simplicité extrême, donnant aux utilisateurs un moyen tout à fait facilité de réaliser une prise de sang, tout en leur assurant une sécurité complète.

Comme il va de soi, l'invention ne se limite pas au seul mode de mise en oeuvre de ce procédé décrit ci-dessus à titre d'exemple, elle embrasse au contraire toutes les variantes sans pour autant sortir du domaine de l'invention.

**Revendications**

1. Dispositif pour éviter les blessures accidentelles dans la manipulation des seringues ou autres systèmes analogues d'injections ou de prises de sang, comprenant une aiguille (2) et un protecteur (3) présentant une rainure (8) dans laquelle vient s'escamoter l'aiguille (2), caractérisé en ce qu'il comporte un manchon droit (4) souple et élastique sur lequel sont montés l'aiguille (2) et le protecteur (3), le manchon (4) et le protecteur étant destinés à être montés fixes sur la partie antérieure d'un support de tube à vide (1) ou de seringue et coopérant entre eux de manière que, lorsqu'une action est exercée sur le manchon (4), il puisse subir un mouvement angulaire-curviligne libérant, l'aiguille (2) de sa position de protection dans la rainure pour l'amener dans une position de pénétration intraveineuse, l'élasticité du manchon (4) étant telle que lorsque ladite action est relâchée, l'aiguille (2) est ramenée dans sa position dans la rainure (8).

2. Dispositif selon la revendication 1, caractérisé en ce que le protecteur (3) est formé par une pièce rigide, en matière plastique, qui comprend un cone ou un filetage destiné à fixer le protecteur à la partie antérieure du support de tubes à vide (1) ou de la seringue.

3. Dispositif selon les revendicaitons 1 ou 2, caractérisé en ce que le manchon est translucide, formant une chambre (11) permettant un contrôle visuel du reflux du sang dans le dispositif se produisant lorsque la pointe de l'aiguille (2) a pénétré dans la veine.

4. Dispositif selon l'une quelconque des revendciations précédentes, caractérisé en ce que l'aiguille intraveineuse (2) est incurvée à sa base.

5. Dispositif selon l'une quelconque des revendicaitons 1 à 3, caractérisé en ce que l'embase (6) de l'aiguille est elles-même incurvée.

**Claims**

1. Device for preventing accidental injuries when using syringes or other analogous systems for making injections or taking samples of blood, comprising a needle 12) and a protector (3), which has a groove (8), into which the needle (2) withdraws, characterised in that it comprises a straight, flexible and resilient sleeve (4), on which are mounted the needle (2) and the protector (3), the sleeve (4) and the protector being intended to be secured on the front portion of a vacuum tube holder (1) or a syringe holder and co-operating with one another so that, when a pressure is exerted upon the sleeve (4), it can make an angular-curvilinear movement which releases the needle (2) from its position of protection in the groove to bring it into an intravenous penetrating position, the resilience of the sleeve (4) being such that, when said pressure is relieved, the needle (2) is returned into its position in the groove (8).

2. Device according to claim 1, characterised in that the protector (3) is formed by a rigid component part, made of plastics material, which comprises a cone or a thread for securing the protector to the front portion of the vacuum tube holder (1) or the syringe holder.

3. Device according to claim 1 or 2, characterised in that the sleeve is translucent, forming a chamber (11) which permits visual control of the reflux of the blood in the device to be effected when the point of the needle (2) has penetrated the vein.

4. Device according to any of the preceding claims, characterised in that the intravenous needle (2) is inwardly curved at its base.

5. Device according to any of claims 1 to 3, characterised in that the base (6) of the needle is itself inwardly curved.

**Patentansprüche**

1. Vorrichtung zum Vermeiden zufälliger Verletzungen beim Umgang mit Spritzen oder anderen ähnlichen Injektionssystemen oder beim Abnehmen von Blut, mit einer Nadel (2) und einer Schutzvorrichtung (3), die eine Nut (8) aufweist, in welche sich die Nadel (2) einlegen kann, gekennzeichnet durch eine biegsame und elastische gerade Hülse (4), an der die Nadel (2) und die Schutzvorrichtung (3) angebracht sind, wobei die Hülse (4) und die Schutzvorrichtung dazu bestimmt sind, am vorderen Teil eines Vakuum-Rohrsupports (1) oder der Spritze befestigt zu werden und derart zusammenwirken, daß die Hülse (4) bei einer darauf ausgeübten Kraft eine winkelförmige, krummlinige Bewegung durchführen kann, durch welche die Nadel (2) aus ihrer in der Nut befindlichen geschützten Stellung in eine ein intravenöses Einstechen gestattende Stellung gebracht wird, wobei die Elastizität der Hülse (4) derart ist, daß die Nadel (2) bei einem Nachlassen der Kraft in ihre in der Nut (8) befindliche Stellung zurückgeführt wird.

2. Vorrichtung nach Anspruch 1, dadurch gekennzeichnet, daß die Schutzvorrichtung (3) aus einem festen, kunststofförmigen Teil gebildet ist, das zum Befestigen der Schutzvorrichtung am vorderen Teil des Vakuum-Rohrsupports (1) oder der Spritze einen Konus oder ein Gewinde aufweist.

3. Vorrichtung nach Anspruch 1 oder 2, dadurch gekennzeichnet, daß die Hülse durchscheinend ist und eine Kammer (11) bildet, die eine visuelle Kontrolle der Rückströmung des Blutes in die Vorrichtung gestattet, welche sich einstellt, wenn die Spitze der Nadel (2) in eine Vene eingestochen ist.

4. Vorrichtung nach einem der vorhergehenden Ansprüche, dadurch gekennzeichnet, daß die intravenöse Nadel (2) an ihrer Basis gekrümmt ist.

5. Vorrichtung nach einem der Ansprüche 1 bis 3, dadurch gekennzeichnet, daß der Sitz (6) der Nadel selbst gekrümmt ist.

FIG.1

FIG.2

FIG.3